(19)

Europäisches
Patentamt
European
Patent Office
Office européen
des brevets

(11) **EP 1 911 529 A1**

(12) **EUROPEAN PATENT APPLICATION**

(43) Date of publication:
**16.04.2008 Bulletin 2008/16**

(51) Int Cl.:
***B06B 1/02*** *(2006.01)*

(21) Application number: **07019918.7**

(22) Date of filing: **11.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **12.10.2006 JP 2006279237**

(71) Applicant: **OLYMPUS MEDICAL SYSTEMS CORP.**
**Shibuya-ku**
**Tokyo 151-0072 (JP)**

(72) Inventors:
• **Matsumoto, Kazuya**
  **Tokyo 151-0072 (JP)**

• **Hasegawa, Mamoru**
  **Tokyo 151-0072 (JP)**
• **Ohta, Ryo**
  **Tokyo 151-0072 (JP)**
• **Adachi, Hideo**
  **Tokyo 151-0072 (JP)**
• **Wakabayashi, Katsuhiro**
  **Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(54) **Ultrasonic transducer, ultrasonic probe, and ultrasonic diagnostic apparatus**

(57) An ultrasonic transducer of a capacitance type includes a pair of electrodes disposed to be opposed to each other and a vibration film including one of the pair of electrodes. The ultrasonic transducer includes n (n is a natural number equal to or larger than 1) air gap layers interposed between the pair of electrodes and m (m is a natural number equal to or larger than 1) insulating layers interposed between the pair of electrodes. In the ultrasonic transducer, a following Formula (1) is satisfied:

$$d_1 \leq \{\Sigma d_n + \Sigma(t_m/K_m)\}/3 \qquad \dots (1)$$

where, $d_n$ indicates a thickness of an nth air gap layer, $d_1$ indicates a thickness of an air gap layer for allowing the vibration film to vibrate, $t_m$ indicates a thickness of an mth insulating layer, and $K_m$ indicates a dielectric constant of the mth insulating layer.

**FIG.6**

EP 1 911 529 A1

**Description**

BACKGROUND OF THE INVENTION

1. Field of the Invention

The present invention relates to a capacitive ultrasonic transducer, an ultrasonic probe, and an ultrasonic diagnostic apparatus.

2. Description of the Related Art

An ultrasonic diagnostic method of irradiating an ultrasound into a body cavity and visualizing and diagnosing a state in the body from an echo signal of the ultrasound is widely used. As one of ultrasonic diagnostic apparatuses used for the ultrasonic diagnostic method, there is an ultrasonic endoscope. In the ultrasonic endoscope, an ultrasonic probe including an ultrasonic transducer is disposed at a distal end of an insertion section led into the body cavity. The ultrasonic transducer has a function of converting an electric signal into an ultrasound to transmit the ultrasound into the body cavity and receiving the ultrasound reflected in the body cavity to convert the ultrasound into an electric signal.

Conventionally, as the ultrasonic transducer, a piezoelectric element such as ceramic piezoelectric material PZT (lead zirconate titanate) has been mainly used. However, in recent years, a Capacitive Micromachined Ultrasonic Transducer (hereinafter referred to as c-MUT) manufactured by using the micromachining technique has been attracting attention.

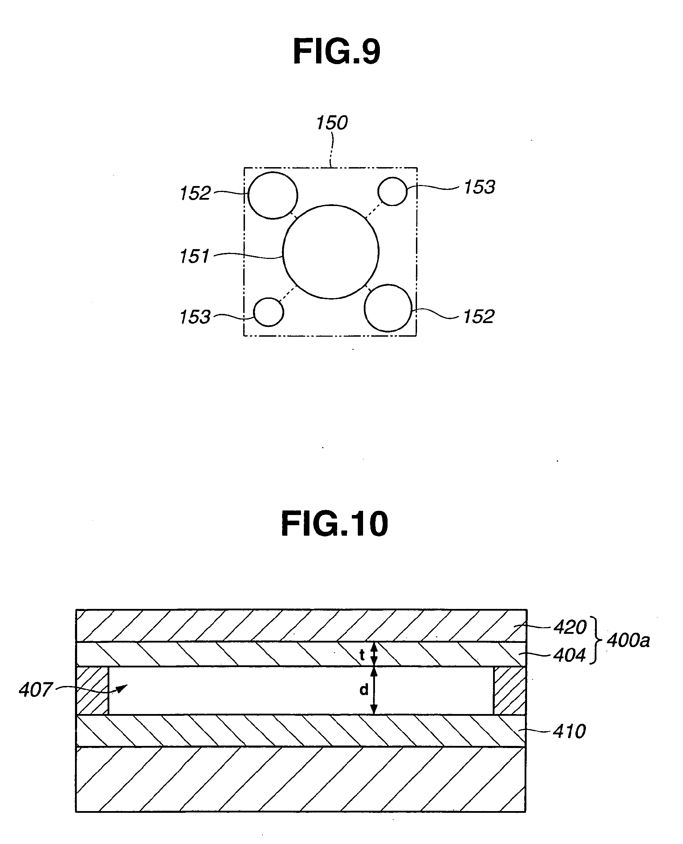

The c-MUT includes, for example, as shown in Fig. 10, a pair of flat electrodes (parallel plate electrodes) 420 and 410 opposed to each other across a cavity 407. The c-MUT transmits and receives an ultrasound according to the vibration of a membrane 400a including one of the electrodes, the upper electrode 420. The c-MUT is disclosed in, for example, United State Patent Application Publication US2005/0219953A1.

In MEMS (Micro Electro Mechanical Systems) of a capacitance type employing parallel plate electrodes such as the c-MUT, it is known that a so-called pull-in phenomenon occurs. The pull-in phenomenon is a phenomenon in which, when a distance between both the electrodes is smaller than a predetermined value, since an electrostatic attraction surpasses a restoring force of a membrane, the membrane 400a including the upper electrode 420 sticks to the other electrode, the lower electrode 410.

A condition under which the pull-in phenomenon occurs is represented by a following equation:

$$xp=(d+t/k)/3 \qquad ... (3)$$

where, xp indicates a displacement of the membrane 400a, d indicates a thickness of the cavity 407, k indicates a dielectric constant of an insulating film 404, and t indicates a thickness of an insulating layer.

The condition under which the pull-in phenomenon occurs is described in, for example, Bozkurt, A., et al., "Theory and analysis of electrode size optimization for capacitive microfabricated ultrasonic transducers", "IEEE TRANSACTIONS ON ULTRASONICS, FERROELECTRICS, AND FREQUENCY CONTROL", VOL. 46, NO. 6, P1364-1374, Nov. 1999, as Equation (25).

When the pull-in phenomenon occurs in the c-MUT, the entire membrane deforms to stick to an electrode opposed to the membrane. Therefore, stresses concentrate in a peripheral portion of the membrane and the pull-in phenomenon occurs, whereby the durable life of the membrane is reduced or a characteristic thereof is changed. The membrane may be destroyed by the pull-in phenomenon.

It is possible to drive the c-MUT under the condition under which the pull-in phenomenon occurs. However, in this case, since the amplitude of the membrane is limited to a fixed value at a driving voltage equal to or higher than a value at which the pull-in phenomenon occurs. Thus, a range in which an output of an ultrasound can be controlled is reduced.

An ultrasonic endoscope of an electronic scanning type includes plural c-MUTs. In general; in the ultrasonic endoscope of the electronic scanning system, for improvement of an image quality, the timing of transmission and the output of a transmission ultrasound are controlled for each of the c-MUTs to control a shape and a direction of an ultrasonic beam to be irradiated. In such an ultrasonic endoscope of the electron scanning system, when the pull-in phenomenon occurs, since a sound pressure of the transmission ultrasound becomes discontinuous, it is difficult to appropriately control the ultrasonic beam. In a state in which the pull-in phenomenon occurs, since the vibration of the membrane is not a sine wave shape, a sound quality falls.

When the c-MUT is driven under a condition under which the pull-in phenomenon does not occur, since a maximum value of the driving voltage is limited, it is inevitable to reduce the output of the ultrasound.

Therefore, in the c-MUT in which the pull-in phenomenon occurs, a characteristic of transmission and reception of the ultrasound is limited.

SUMMARY OF THE INVENTION

An ultrasonic transducer according to the present invention is an ultrasonic transducer of a capacitance type including a pair of electrodes disposed to be opposed to each other and a vibration film including one of the pair of electrodes. The ultrasonic transducer includes n (n is a natural number equal to or larger than 1) air gap layers interposed between the pair of electrodes and m (m is a natural number equal to or larger than 1) insulating layers interposed between the pair of electrodes. In the ultrasonic transducer, a following Formula (1) is satisfied:

$$d_1 \leq \{\Sigma d_n + \Sigma(t_m/K_m)\}/3 \qquad \dots (1)$$

where, $d_n$ indicates a thickness of an nth air gap layer, $d_1$ indicates a thickness of an air gap layer for allowing the vibration film to vibrate, $t_m$ indicates a thickness of an mth insulating layer, and $K_m$ indicates a dielectric constant of the mth insulating layer.

The above and other objects, features and advantages of the invention will become more clearly understood form the following description referring to the accompanying drawings.

BRIEF DESCRIPTION OF THE DRAWINGS

Fig. 1 is a diagram for explaining the schematic structure of an ultrasonic endoscope;
Fig. 2 is a perspective view showing the structure of a distal end portion of the ultrasonic endoscope;
Fig. 3 is a perspective view of a transducer array;
Fig. 4 is a top view of transducer units viewed from a transmitting and receiving direction of an ultrasound;
Fig. 5 is a top view of a transducer cell;
Fig. 6 is a cross-sectional view taken along line VI-VI in Fig. 5;
Fig. 7 is a cross-sectional view of a transducer cell according to a second embodiment of the present invention;
Fig. 8 is a cross-sectional view of a transducer cell according to a third embodiment of the present invention;
Fig. 9 is a top view of an transducer element according to a fourth embodiment of the present invention; and
Fig. 10 is a cross-sectional view for explaining the structure of a conventional oscillator cell.

DETAILED DESCRIPTION OF THE PREFERRED EMBODIMENTS

The present invention provides a capacitive ultrasonic transducer including a pair of electrodes disposed to be opposed to each other and a vibration film including one of the pair of electrodes. The ultrasonic transducer includes n (n is a natural number equal to or larger than 1) air gap layers interposed between the pair of electrodes and m (m is a natural number equal to or larger than 1) insulating layers interposed between the pair of electrodes. In the ultrasonic transducer, a following Formula (1) is satisfied:

$$d_1 \leq \{\Sigma d_n + \Sigma(t_m/K_m)\}/3 \qquad \dots (1)$$

where, $d_n$ indicates a thickness of an nth air gap layer, $d_1$ indicates a thickness of an air gap layer for allowing the vibration film to vibrate, $t_m$ indicates a thickness of an mth insulating layer, and $K_m$ indicates a dielectric constant of the mth insulating layer. In a following explanation, the air gap layer for allowing the vibration film to vibrate is described as "cavity" as well.

(First Embodiment)

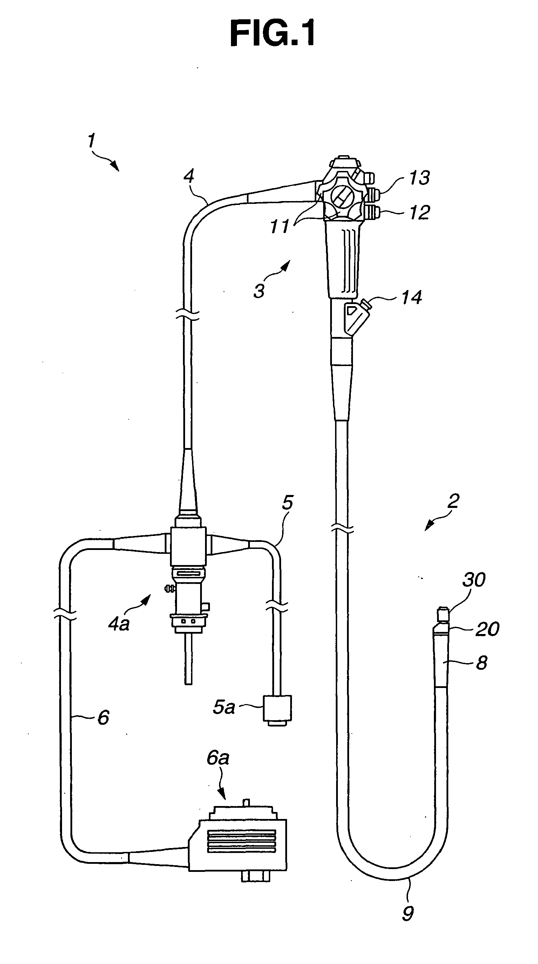

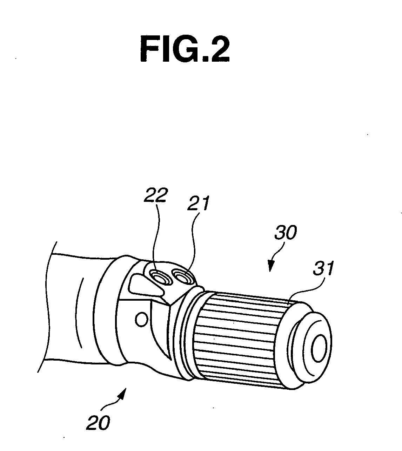

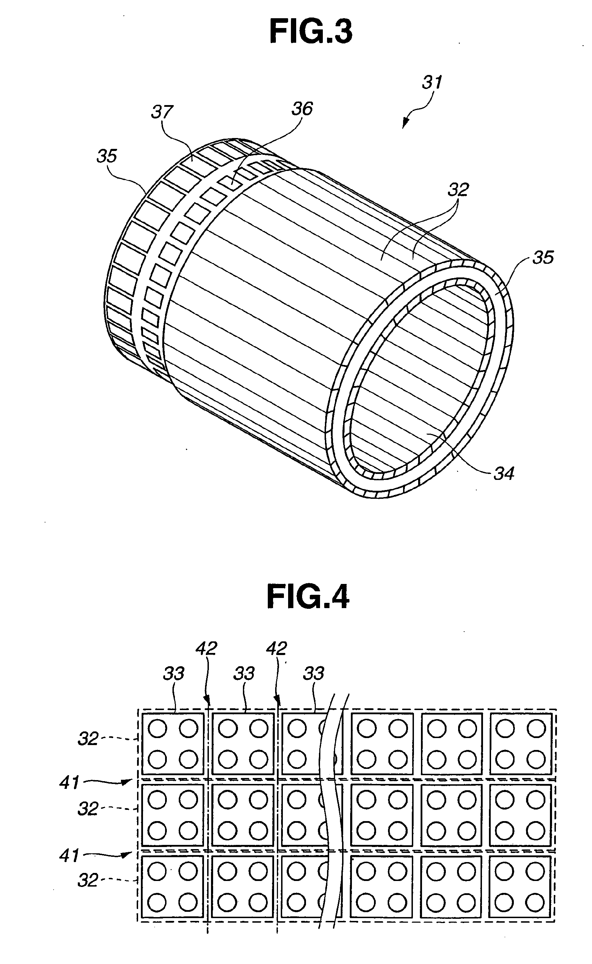

A first embodiment of the present invention is explained below with reference to Figs. 1 to 6 and Fig. 10. In respective figures used in a following explanation, scales are varied for respective members in order to show the members in sizes recognizable on the figures. Fig. 1 is a diagram for explaining the schematic structure of an ultrasonic endoscope. Fig. 2 is a perspective view showing the structure of a distal end portion of the ultrasonic endoscope. Fig. 3 is a perspective view of a transducer array. Fig. 4 is a top view of transducer units viewed from a transmitting and receiving direction of an ultrasound.

As shown in Fig. 1, an ultrasonic endoscope 1 as an ultrasonic diagnostic apparatus according to the present embodiment mainly includes a slender insertion section 2 led into a body cavity, an operation section 3 located at a proximal end of the insertion section 2, and a universal cord 4 extending from a side of the operation section 3.

An endoscope connector 4a connected to a not-shown light source device is provided at a proximal end of the universal cord 4. From the endoscope connector 4a, an electric cable 5 detachably connected to a not-shown camera control unit via an electric connector 5a and an ultrasonic cable 6 detachably connected to a not-shown ultrasonic observation apparatus via an ultrasonic connector 6a are extended.

The insertion section 2 is constituted by connecting, from a distal end side thereof, in order, a distal end rigid portion 20 formed of a hard resin member, a bendable bending portion 8 located at a rear end of the distal end rigid portion 20, and a thin and long flexible tube portion 9 having flexibility located at a rear end of the bending portion 8 and leading to a distal end portion of the operation section 3. An ultrasound transmitting and receiving section 30 for transmitting and receiving an ultrasound, which is described later in detail, is provided on a distal end side of the distal end rigid portion 20.

In the operation section 3, an angle knob 11 for controlling to bend the bending portion 8 in a desired direction, an air-supply/water-supply button 12 for performing air supply and water supply operation, a suction button 13 for performing suction operation, and a treatment-instrument insertion port 14 serving as an entrance of a treatment instrument led into the body cavity, and the like are provided.

As shown in Fig. 2, in the distal end rigid portion 20, an illumination lens (not shown) constituting an illumination optical section that irradiates illumination light on an observation region, an object lens 21 constituting an observation optical section that captures an optical image of the observation region, a suction and forceps port 22 serving as an opening for sucking a cut region and projecting the treatment instrument, an air-supply and water-supply port (not shown) for performing air supply and water supply are provided.

The ultrasound transmitting and receiving section 30 provided at a distal end of the distal end rigid portion 20 includes, as shown in Fig. 3, a transducer array 31, a driving circuit 34, and an FPC 35. The FPC 35 is a wiring board (a flexible wiring board) that has flexibility and on both sides of which mounting surfaces are formed. In the ultrasound transmitting and receiving section 30, the FPC 35 is disposed to be wound around in a substantially cylindrical shape with an axis substantially parallel to an insertion axis of the distal end rigid portion 20 as a center axis.

The transducer array 31, which is as a two-dimensional ultrasonic transducer array, is provided on an outer peripheral surface of the cylindrical FPC 35. The transducer array 31 includes plural transducer units 32 (ultrasonic probes) arrayed in a circumferential direction on the outer peripheral surface of the FPC 35. The transducer units 32 have a substantially rectangular shape viewed from a normal direction of the outer peripheral surface of the FPC 35. The transducer units 32 are arrayed at equal intervals on the outer peripheral surface of the cylindrical FPC 35 with a latitudinal direction thereof set in the circumferential direction. The transducer array 31 includes, for example, several tens to several hundreds transducer units 32. The transducer array 31 according to the present embodiment includes 128 pieces of transducer units 32.

As shown in Fig. 4, each of the transducer units 32 is constituted by arraying plural transducer elements 33. In the present embodiment, the transducer elements 33 have a substantially square shape viewed from a normal direction of the outer peripheral surface of the FPC 35. The transducer elements 33 are arrayed one-dimensionally in a longitudinal direction of the transducer unit 32. In the present embodiment, the transducer unit 32 includes sixty-four transducer elements 33.

Each of the transducer elements 33 includes plural transducer cells 100 serving as ultrasonic transducers according to the present embodiment described in detail later. In an identical transducer element 33, all the transducer cells 100 are electrically connected in parallel. When a driving signal from the ultrasonic observation apparatus is inputted to the transducer element 33, the transducer element 33 simultaneously transmits ultrasounds of the same phase. In other words, in the present embodiment, one transducer element 33 is a minimum driving unit for transmitting and receiving an ultrasound. In the present embodiment, the transducer element 33 includes four transducer cells 100.

An transducer unit boundary groove 41 serving as a groove for sectioning the respective transducer units 32 is formed between the transducer units 32 adjacent to each other. A transducer element boundary groove 42 serving as a groove for sectioning the respective transducer elements 33 is formed between the transducer elements 33. By providing the grooves in an outer periphery of the transducer elements 33 as the minimum driving unit in this way, it is possible to reduce crosstalk between the transducer elements 33 adjacent to each other.

The transducer element 33 transmits an ultrasound in a normal direction of the mounting surfaces of the FPC 35, i.e., outward in a radial direction of the cylindrical FPC 35. Therefore, the transducer units 32 constituted by arraying the transducer elements 33 one-dimensionally constitutes a one-dimensional ultrasonic transducer array. The transducer array 31, which is a two-dimensional ultrasonic transducer array, is constituted by arraying a plurality of the transducer units 32.

On the other hand, plural driving circuits 34 are mounted on an inner peripheral surface of the cylindrical FPC 35, i.e., on the mounting surface on the opposite side of the mounting surface on which the transducer array 31 is mounted. The driving circuits 34 have electric circuits such as pulsars and selectors for driving the transducer elements 33 and are electrically connected to the respective transducer elements 33.

The driving circuits 34 are also electrically connected to plural signal electrodes 36 and ground electrodes 37 formed on the outer peripheral surface of the cylindrical FPC 35. The signal electrodes 36 are shown as one electrode in Fig. 3. However, the signal electrodes 36 are divided according to the number of the transducer elements 33. One signal electrode 36 is disposed for one transducer element 33.

A coaxial cable inserted through the ultrasonic cable 6 and electrically connected to the ultrasonic connector 6a at one end is electrically connected to the signal electrodes 36 and the ground electrodes 37 at the other end. Therefore, the driving circuit 34 is electrically connected to the ultrasonic observation apparatus.

The ultrasound transmitting and receiving section 30 having the structure described above simultaneously or alternately performs, using the transducer array 31, which is the two-dimensional ultrasonic transducer array disposed on the outer peripheral surface of the cylindrical FPC 35, so-called electronic radial scanning for radially transmitting and receiving an ultrasound on a plane substantially orthogonal to the insertion axis of the distal end rigid portion 20, and so-called electronic sector scanning for radially transmitting and receiving an ultrasound on a plane including the insertion axis of the distal end rigid portion 20. In other words, the ultrasonic endoscope 1 according to the present embodiment is capable of performing three-dimensional ultrasonic scanning in the body cavity.

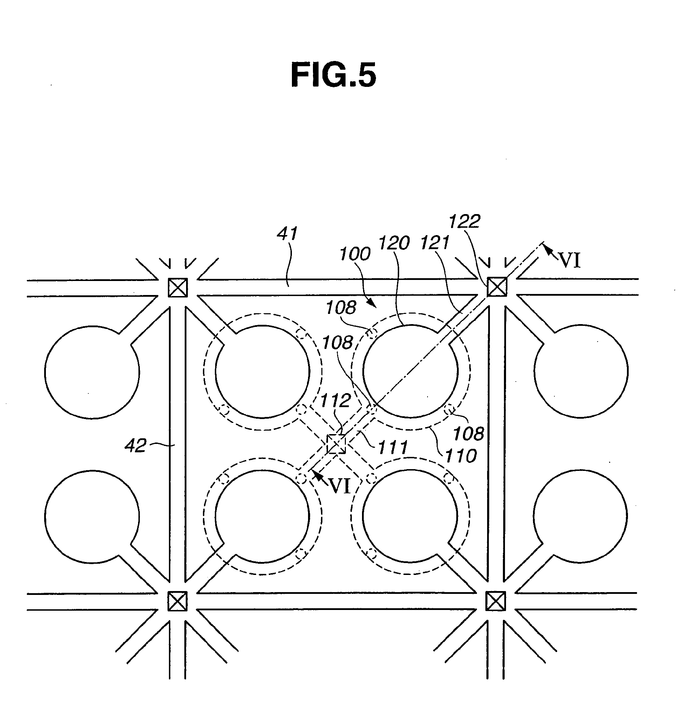

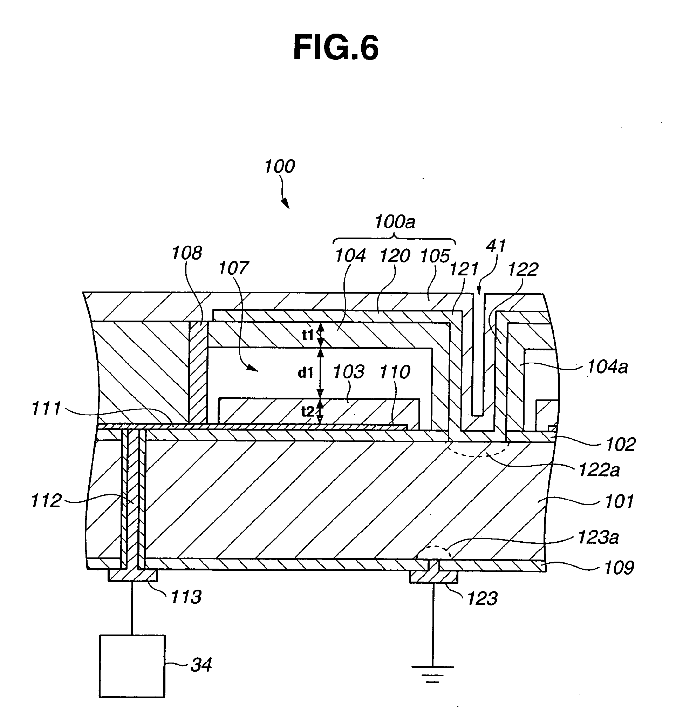

The structure of the transducer cell 100 as a capacitance type ultrasonic transducer according to the present embodiment is explained below. Fig. 5 is a top view of the transducer cell 100 constituting the transducer element 33. Fig. 6 is a cross-sectional view taken along line VI-VI in Fig. 5.

The transducer cell 100 according to the present embodiment belongs to a technical field of so-called MEMS (Micro Electro Mechanical Systems). The transducer cell 100 is a capacitive ultrasonic transducer formed on a silicon substrate 101 by the micromachining technique and is referred to as a c-MUT (Capacitive Micromachined Ultrasonic Transducer). In the present embodiment, the transducer cell 100 includes a parallel plate electrode formed by using a semiconductor process and has a layered structure.

In a following explanation of the layered structure, concerning a positional relation among respective layers, a direction away from the surface of the silicon substrate 101 in a normal direction is set as an upward direction. For example, in the cross-sectional view in Fig. 6, an upper electrode 120 is disposed above a lower electrode 110. Thicknesses of the respective layers indicate dimensions of the respective layers in a direction parallel to a normal of a surface of the silicon substrate 101. In a following explanation, for convenience of explanation, among surfaces of the silicon substrate 101, a surface on which the transducer 100 is formed is referred to as a cell formation surface and a surface on the opposite side of the surface on which the transducer cell 100 is formed is referred to as a rear surface.

The transducer cell 100 includes the lower electrode 110 (a first electrode) and the upper electrode 120 (a second electrode), which are a pair of parallel plate electrodes opposed to each other via a substantially cylindrical cavity 107. The transducer cell 100 transmits and receives an ultrasound according to a change in a distance between both the electrodes, i.e., the vibration of a membrane 100a (a vibration film), which is a film-like structure having elasticity, including the upper electrode 120. In other words, the cavity 107 forms an air gap layer for allowing the membrane 100a to vibrate.

The structure of the transducer cell 100 according to the present invention is explained below in detail.

The silicon substrate 101 is formed of a low-resistivity silicon wafer having conductivity. On both surfaces of the silicon substrate 101, a first insulating film 102 and a rear-surface insulating film 109, which are silicon oxide films having an electric insulating property, are formed, respectively. The first insulating film 102 and the rear-surface insulating film 109 are high-temperature oxide films formed by thermally oxidizing the silicon substrate 101.

On a cell formation surface of the silicon substrate 101, i.e., on the first insulating film 102, the lower electrode 110, which is a conductive layer, is formed in a substantially circular shape viewed from above. The lower electrode 110 is formed by subjecting Mo (molybdenum) to film formation and pattering according to sputtering. Four lower electrodes 110 adjacent to one another viewed from above are electrically connected by a lower electrode wiring 111 of a substantial X shape.

It is desirable that a material for forming the lower electrode 110, which is a lower layer section of the layered structure and formed on the silicon oxide film, is, other than Mo, refractory metal such as W (tungsten), Ti (titanium), and Ta (tantalum) and alloys of the metal. However, the material is not limited to the metal and the alloys and may be Al (aluminum), Cu (copper), and the like as long as it is possible to prevent high-temperature thermal processing in a manufacturing process after the formation. The lower electrode 110 may have a multilayer structure in which two or more kinds of conductive materials are stacked.

In a crossing portion of the lower electrode wiring 111 of the substantial X shape viewed from above, a through wafer inter connect 112 formed to pierce through the silicon substrate 101 is provided. The through wafer inter connect 112 is electrically insulated from the silicon substrate 101 and is electrically connected to a signal electrode pad 113 formed on the rear-surface insulating film 109.

In other words, the lower electrode 110 is electrically connected to the signal electrode pad 113 formed on the rear surface of the silicon substrate 101 via the lower electrode wiring 111 and the through wafer inter connect 112.

A second insulating film 103 having an electric insulating property is formed on the lower electrode 110 to cover the lower electrode 110. The second insulating film 103 is a silicon oxide film in the present embodiment and is formed by the plasma CVD method. The second insulating film 103 may be formed of a silicon nitride film, hafnium nitride (HfN), hafnium oxide nitride (HfON), and the like.

On the second insulating film 103, a third insulating film 104 having an electric insulating property is formed across the cavity 107. The third insulating film 104 is a silicon oxide film in the present embodiment and is formed by the plasma CVD method. The third insulating film 104 may be a silicon nitride film.

The cavity 107, which is an air gap layer in a closed first layer in an atmospheric pressure, compressed, or decompressed state, is formed between the second insulating film 103 and the third insulating film 104. The decompressed state indicates a state in which a pressure is lower than the atmospheric pressure and includes a so-called vacuum state. The cavity 107 has a substantially cylindrical shape and is provided substantially concentrically with the lower electrode 110 viewed from above.

In the present embodiment, the cavity 107 is formed by sacrificial layer etching, which is a well known technique. Sacrificial layer removing holes for causing the inside of the cavity 107 and a layer above the third insulating film 104 to communicate with each other, which are used during the sacrificial layer etching, are sealed by plugs 108. The holes for removed of sacrificial layer are formed in three places in an outer periphery of the cavity 107. The cavity 107 may be formed by a method of wafer bonding after microprocessing.

The upper electrode 120, which is a conductive layer of a substantially circular shape viewed from above is formed on the third insulating film 104. The upper electrode 120 is provided substantially concentrically with the lower electrode 110 viewed from above, i.e., in a position opposed to the lower electrode 110. In the present embodiment, the upper electrode 120 is formed by subjecting Al to film formation and patterning according to sputtering.

Four upper electrodes 120 adjacent to one another viewed from above are electrically connected by an upper electrode wiring 121 of a substantial X shape. The upper electrode wiring 121 is disposed in a position not overlapping the lower electrode wiring 111 viewed from above. By arranging the lower electrode wiring 111 and the upper electrode wiring 121 not to overlap each other in this way, it is possible to prevent generation of a parasitic capacitance in a wiring section.

A material forming the upper electrode 120 only has to be, other than Al, materials having conductivity such as Cu, W, Ti, and Ta. The upper electrode 120 may have a multilayer structure in which two or more kinds of conductive materials are stacked.

A crossing portion of the upper electrode wiring 121 of the substantial X shape viewed from above is formed on the transducer unit boundary groove 41. The transducer unit boundary groove 41 is formed at depth for piercing through a membrane supporting section 104a of the second insulating film 104 and reaching the silicon substrate 101. In the transducer unit boundary groove 41, a through electrode 122 is formed in a process identical with that for the upper electrode 120 and the upper electrode wiring 121. The through electrode 122 is electrically connected to the silicon substrate 101 via an ohmic contact area 122a.

A ground electrode pad 123 is formed on the rear-surface insulating film 109. The ground electrode pad 123 is electrically connected to the silicon substrate 101 via an ohmic contact area 123a.

In other words, the upper electrode 120 is electrically connected to the ground electrode pad 123 formed on the rear surface of the silicon substrate 101 via the upper electrode wiring 121, the through electrode 122, and the silicon substrate 101.

On the upper electrode 120, a protection film 105 having an electric insulating property is formed. In the present embodiment, the protection film 105 is a silicon nitride film and is formed by the plasma CVD method. The protection film 105 may be a silicon oxide film. The protection film 105 may be formed of, other than the silicon nitride, a silicon oxide film, hafnium nitride (HfN), hafnium oxide nitride (HfON), and the like. In particular, HfN and HfON are preferable as a protection film because a high-density film is obtained.

Although not shown in the figure, on the protection film 105, a film formed of paraxylylene resin or the like having water resistance, chemical resistance, and the like and excellent in biocompatibility and an electric insulating property may be formed.

The transducer element 31 including the transducer cell 100 having the structure described above is mounted on the FPC 35 by a publicly known method such as solder joining, anisotropic conductive film joining, or ultrasonic joining. Therefore, the transducer cell 100 is electrically connected to the driving circuit 34 mounted on the opposite side of the FPC 35 via the signal electrode pad 113 and the ground electrode pad 123.

In the transducer cell 100 having the structure described above, a portion corresponding to an area on the cavity 107 in the third insulating film 104, the upper electrode 120, and the protection film 105 constitutes the membrane 100a serving as a vibration film. The cavity 107 constitutes an air gap layer for allowing the membrane 100a to vibrate.

Between the lower electrode 110 and the upper electrode 120, a layer structure including the second insulating film 103 (an insulating layer in a second layer), the cavity 107 (the air gap layer in the first layer), and the third insulating film 104 (the insulating layer in the first layer) is interposed. These layers are formed to have thicknesses $t_2$, $d_1$, and $t_1$, respectively.

In general, dimensions of the respective layers of the cavity 107, the second insulating film 103, and the third insulating film 104 are determined on the basis of conditions necessary for the transducer cell 100 such as a frequency and an output of an ultrasound to be transmitted and received and a dielectric voltage. In the present embodiment, thicknesses of the respective layers are determined to satisfy a condition of a following Formula (1) in addition to the conditions described above.

$$d_1 \leq \{\Sigma d_n + \Sigma(t_m/K_m)\}/3 \qquad \dots (1)$$

where, n and m are natural numbers equal to or larger than 1, $d_n$ indicates a thickness of the air gap layer, $d_1$ indicates a thickness of the air gap layer for allowing the membrane 100a (the vibration film) to vibrate, $t_m$ indicates a thickness of the insulating layer, and $K_m$ indicates a dielectric constant of the insulating layer.

Formula (1) is newly derived by generalizing the conditional formula for occurrence of the pull-in phenomenon in the Non-Patent Document 1 to be applicable to wider range of conditions and changing the equal sign to the inequality sign to prevent the pull-in phenomenon from occurring. When Formula (1) is satisfied, the pull-in phenomenon does not occur in the transducer cell 100. In other words, Formula (1) indicates a condition under which, even when the amplitude of the membrane 100a is a maximum amplitude, i.e., $d_1$, an electrostatic attraction between the electrodes does not exceed a restoring force of the membrane 100a.

It is goes without saying that, as shown in Fig. 10, when only one layer of an insulating film is present between the lower electrode 110 and the upper electrode 120 or when three or more layers are present, Formula (1) is applicable.

For example, in the transducer cell 100 according to the present embodiment, when a thickness $d_1$ of the cavity 107 is decided as 0.1 $\mu$m from an output value of an ultrasound, a following formula is applied.

$$0.1 \leq (0.1 + t_1/K_1 + t_2/K_2)/3$$

Since the second insulating film 103 and the third insulating film 104 are formed of a silicon oxide film ($SiO_2$), $K_1 = K_2 \approx 4$. Therefore, a thicknesses of the second insulating film 103 and the third insulating film 104 are determined to satisfy a following formula:

$$(t_1 + t_2) \geq 0.8 \ [\mu m]$$

The transducer cell 100 according to the present embodiment having the structure described above has the structure in which the pull-in phenomenon does not occur. Thus, the degradation in an ultrasound quality and in an output of an ultrasound due to the pull-in phenomenon do not occur. Since the pull-in phenomenon does not occur in the transducer cell 100, there is no limit in a driving voltage to be applied and an output of an ultrasound is not limited.

Therefore, according to the present embodiment, it is possible to realize an ultrasonic transducer having a satisfactory characteristic. In an ultrasonic endoscope including the ultrasonic transducer according to the present embodiment, it is possible to obtain a satisfactory ultrasonic diagnostic image having a high quality.

In the structure described above, the transducer cell 100 including the lower electrode 110, the upper electrode 120, and the cavity 107 has a substantially circular shape viewed from above. However, the shape of these members is not limited to the substantially circular shape and may be, for example, a regular hexagonal shape and a rectangular shape.

In the structure described above, the silicon substrate 101 is formed of a conductive material in order to have a function of a wiring. However, the silicon substrate 101 may be formed of an insulating material if an alternative wiring is separately provided. In other words, the transducer cell 100 may be formed on a substrate formed not only of silicon but also of quartz, glass, sapphire, ceramic, and the like or may be formed on a substrate formed of a polymer such as polyimide.

In the present embodiment, the transducer cell 100 is electrically connected to the driving circuit via the electrode pad provided on the rear surface of the silicon substrate 101. However, the transducer cell 100 and the driving circuit may be electrically connected via, for example, an electrode and a bonding wire provided on a cell formation surface of the silicon substrate 101.

(Second Embodiment)

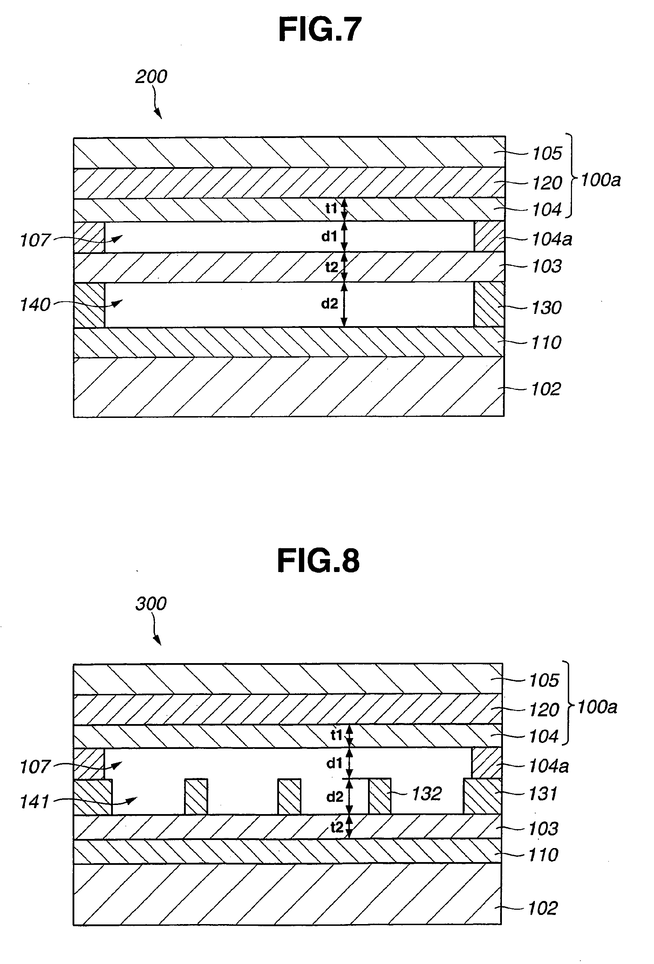

A second embodiment of the present invention is explained below with reference to the drawings. Fig. 7 is a cross-sectional view schematically showing a layered structure of a transducer cell. The transducer cell according to the second embodiment is different from the transducer cell according to the first embodiment only in a layered structure in which an air gap layer is added between an upper electrode and a lower electrode. Therefore, only the difference is explained below. Components same as those in the first embodiment are denoted by the identical reference numerals and signs and explanation of the components is omitted as appropriate.

As shown in Fig. 7, in a transducer cell 200 according to the present embodiment, an air gap layer 140 (an air gap layer in a second layer), which is a space having a thickness $d_2$, is formed between the lower electrode 110 and the second insulating film 103. The air gap layer 140 is formed by providing a spacer 130 including an insulating film having a thickness $d_2$ on the lower electrode 110. The air gap layer 140 is in an atmospheric pressure, compressed, or decompressed state. In the present embodiment, the membrane 100a vibrates in the cavity 107.

In the transducer cell 200 according to the present embodiment, thicknesses of respective layers are determined to satisfy the condition of Formula (1).

For example, in the transducer cell 200 according to the present embodiment, when a thickness $d_1$ of the cavity 107 is decided as 0.1 μm from an output value of an ultrasound as in the first embodiment, a following formula is applied.

$$0.1 \leq (0.1 + d_2 + t_1/K_1 + t_2/K_2)/3$$

Since the second insulating film 103 and the third insulating film 104 are formed of $SiO_2$, $K_1 = K_2 \approx 4$. Therefore, when $d_2$ is decided as 0.1 μm, thicknesses of the second insulating film 103 and the third insulating film 104 are determined to satisfy a following formula:

$$(t_1 + t_2) \geq 0.4[\mu m]$$

In other words, while setting the thickness of the cavity 107, i.e., the amplitude of the membrane to a value equivalent to that in the first embodiment, a distance between the lower electrode 110 and the second insulating film 103 can be reduced by 0.3 μm.

As described above, according to the present embodiment, it is possible to reduce a thickness of the transducer cell 200 by providing the air gap layer 140. Therefore, it is possible to reduce a size of an ultrasonic endoscope including the transducer cell 200. It goes without saying that the second embodiment has effects equivalent to those in the first embodiment.

Air gap layers may be provided in plural places, not limited to the case in the present embodiment. For example, air gap layers having a thickness $d_3$ may be provided in the membrane 100a as air gap layers in a third layer.

(Third Embodiment)

A third embodiment according to the present invention is explained below with reference to the drawings. Fig. 8 is a cross-sectional view schematically showing a layered structure of a transducer cell. The transducer cell according to the third embodiment is different from the transducer cell according to the first embodiment in a layered structure in which an air gap layer is added between an upper electrode and a lower electrode. Therefore, only the difference is explained below. Components same as those in the first embodiment are denoted by the identical reference numerals and signs and explanation of the components is omitted as appropriate.

As shown in Fig. 8, in a transducer cell 300 according to the present embodiment, an air gap layer 141 (an air gap layer in a second layer), which is a space having thickness $d_2$, is formed between the second insulating film 103 and the cavity 107. The cavity 107 and the air gap layer 141 are formed in a connected state without a structure for physically sectioning the cavity 107 and the air gap layer 141. In the present embodiment, an air gap portion having thickness $d_1$ provided for allowing the membrane 100a is defined as the cavity 107. On the other hand, the air gap layer 141 is formed by providing a spacer 131 formed of an insulating film having thickness $d_2$ on the second insulating film 103.

In the air gap layer 141, plural posts 132, which are columnar projections having height $d_2$, are discretely disposed. The posts 132 are formed in a process identical to that for the spacer 131 and are formed of an insulating film. The air gap layer 141 is in an atmospheric pressure, compressed, or vacuum state.

In the transducer cell 300, thicknesses of respective layers are determined to satisfy the condition of Formula (1) as in the first and second embodiments.

In the transducer cell 300 having the structure described above, since the posts 132 are provided, it is possible to reduce an area of the membrane 100a in contact with the structure on the lower electrode 100 side when the amplitude of the membrane 100a is the maximum. Therefore, the membrane 100a does not stick to the lower electrode 110.

Since the air gap layer 141 communicating with the cavity 107 is formed, when the inside of the cavity 107 is in a state other than the vacuum, for example, in the atmospheric pressure state, it is possible to reduce a ratio of compression of the atmosphere in the cavity 107 due to the vibration of the membrane 100a. Consequently, it is possible to reduce the resistance for compressing the atmosphere in the cavity 107, which is generated when the membrane 100a vibrates, and it is possible to more efficiently generate an ultrasound having a high output.

Therefore, the transducer cell 300 according to the present embodiment is capable of more efficiently and stably outputting an ultrasound. It goes without saying that the third embodiment has effects same as those in the second embodiment.

A position where the air gap layer is disposed and the number of air gap layers are not limited to those in the present embodiment. For example, posts may be formed on the membrane 100a side, i.e., in a layer below the third insulating film 104. Plural air gap layers may be formed.

A distal end shape of the posts may be a substantially semicircular shape, a taper shape, and the like.

(Fourth Embodiment)

A fourth embodiment of the present invention is explained below with reference to the drawings. Fig. 9 is a top view schematically showing an arrangement of transducer cells according to the fourth embodiment. The fourth embodiment is different from the first embodiment only in a shape and an arrangement of transducer cells. Therefore, only the difference is explained below. Components same as those in the first embodiment are denoted by the identical reference numerals and signs and explanation of the components is omitted.

In the first embodiment, all the transducer cells 100 have the identical shape. However, in the present embodiment, as shown in Fig. 9, a transducer element 150 includes plural transducer cells 151, 152, and 153 having different shapes.

The shape of transducer cells indicates a shape defined by a diameter of a substantially columnar cavity, a thickness of the cavity, a thickness of a membrane, and the like. The shape of transducer cells is an element that determines a characteristic of an ultrasound transmitted and received by the transducer cells.

In the present embodiment, at least a part of the plural transducer cells 151, 152, and 153 having different shapes are formed to satisfy Formula (1). Therefore, effects same as those in the first embodiment are obtained.

In the present embodiment, an identical driving signal is inputted to the plural transducer cells 151, 152, and 153 having different shapes. Therefore, ultrasounds having different frequencies and outputs are transmitted from the transducer cells 151, 152, and 153, respectively. This makes it possible to expand a frequency band of ultrasounds transmitted by the transducer element 150.

A part of plural transducer elements constituting a transducer unit may include the transducer element 150 according to the present embodiment shown in Fig. 9. Consequently, the transducer unit is capable of transmitting and receiving ultrasounds having different characteristics. In an ultrasonic endoscope including the transducer element 150 or the transducer unit capable of transmitting and receiving ultrasounds having plural different characteristics, it is possible to obtain an ultrasonic tomogram formed by ultrasounds in different bands and it is possible to observe the inside of a body cavity in detail.

The present invention is not limited to the embodiments described above. The present invention can be appropriately changed without departing from the spirit and the idea of the present invention that can be read from the claims and the entire specification. An ultrasonic transducer and an ultrasonic endoscope involving such a change are included in the technical scope of the present invention.

For example, the ultrasonic endoscope according to the embodiments is explained as an ultrasonic endoscope that performs the electronic radial scanning and sector scanning. However, a scanning system is not limited to these. The ultrasonic endoscope may adopt linear scanning, convex scanning, and the like or may adopt a mechanical scanning system. The ultrasound transmitting and receiving section may have a one-dimensional array in which plural transducer elements are arrayed one-dimensionally.

The present invention belongs to a technical field including not only an endoscope including an ultrasound transmitting and receiving section at a distal end thereof but also a so-called ultrasonic probe in general that is led into a body cavity regardless of whether the ultrasonic probe is operated by wire or by radio. For example, the present invention may relate to an ultrasonic probe that is inserted through a treatment instrument insertion hole of an endoscope and led into a body cavity.

**Claims**

1. An ultrasonic transducer of a capacitance type comprising:

   a pair of electrodes disposed to be opposed to each other; and
   a vibration film including one of the pair of electrodes, wherein
   the ultrasonic transducer includes:

   n (n is a natural number equal to or larger than 1) air gap layers interposed between the pair of electrodes; and
   m (m is a natural number equal to or larger than 1) insulating layers interposed between the pair of electrodes, and
   a following Formula (1) is satisfied:

$$d_1 \leq \{\Sigma d_n + \Sigma(t_m/K_m)\}/3 \qquad \ldots (1)$$

   where, $d_n$ indicates a thickness of an nth air gap layer, $d_1$ indicates a thickness of an air gap layer for allowing the vibration film to vibrate, $t_m$ indicates a thickness of an mth insulating layer, and $K_m$ indicates a dielectric constant of the mth insulating layer.

2. The ultrasonic transducer according to claim 1, wherein
   the ultrasonic transducer has two or more of the air gap layers, and
   one or more projections are formed of an insulating material is disposed in at least one of the air gap layers.

3. An ultrasonic probe comprising the ultrasonic transducer according to claim 1.

4. An ultrasonic probe comprising the ultrasonic transducer according to claim 2.

5. An ultrasonic diagnostic apparatus comprising the ultrasonic transducer according to claim 1.

6. An ultrasonic diagnostic apparatus comprising the ultrasonic transducer according to claim 2.

# FIG.1

# FIG.2

# FIG.3

# FIG.4

# FIG.5

# FIG.6

# FIG.7

200

105
120 } 100a
104

t1
d1 — 104a
107 — t2 — 103
140 — d2 — 130
— 110
— 102

# FIG.8

300

105
120 } 100a
104

t1
107 — d1 — 104a
141 — d2 — 132 — 131
t2 — 103
— 110
— 102

# FIG.9

# FIG.10

**European Patent Office**

# EUROPEAN SEARCH REPORT

Application Number

EP 07 01 9918

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | BOZKURT A ET AL: "THEORY AND ANALYSIS OF ELECTRODE SIZE OPTIMIZATION FOR CAPACITIVE MICROFABRICATED ULTRASONIC TRANSDUCERS" IEEE TRANSACTIONS ON ULTRASONICS, FERROELECTRICS AND FREQUENCY CONTROL, IEEE SERVICE CENTER, PISCATAWAY, NJ, US, vol. 46, no. 6, November 1999 (1999-11), pages 1364-1374, XP000913363 ISSN: 0885-3010 * the whole document * | 1-6 | INV. B06B1/02 |
| A | US 2005/234342 A1 (BAYRAM BARIS [US] ET AL BAYRAM BARIS [US] ET AL) 20 October 2005 (2005-10-20) * paragraphs [0003], [0033], [0041] * * abstract; figures 1,4 * | 1 | |
| A | US 2005/228285 A1 (HUANG YONGLI [US] ET AL) 13 October 2005 (2005-10-13) * paragraphs [0003], [0017], [0020], [0021] * * abstract; figures 1,2,4-6 * | 1,2 | TECHNICAL FIELDS SEARCHED (IPC) B06B |

The present search report has been drawn up for all claims

| Place of search | Date of completion of the search | Examiner |
|---|---|---|
| Munich | 20 February 2008 | Passier, Martinus |

EPO FORM 1503 03.82 (P04C01)

## ANNEX TO THE EUROPEAN SEARCH REPORT
## ON EUROPEAN PATENT APPLICATION NO.

EP 07 01 9918

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2008

| Patent document cited in search report | Publication date | Patent family member(s) | Publication date |
|---|---|---|---|
| US 2005234342 A1 | 20-10-2005 | NONE | |
| US 2005228285 A1 | 13-10-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050219953 A1

**Non-patent literature cited in the description**

- **BOZKURT, A et al.** Theory and analysis of electrode size optimization for capacitive microfabricated ultrasonic transducers. *IEEE TRANSACTIONS ON ULTRASONICS, FERROELECTRICS, AND FREQUENCY CONTROL,* November 1999, vol. 46 (6), 1364-1374